# EUROPEAN PATENT APPLICATION

(11) **EP 4 474 345 A1**
(43) Date of publication of application: **11.12.2024**
(21) Application number: 23774975.9
(22) Date of filing: 22.03.2023
(51) Int. Cl.: C01B 21/064, A61K 8/19

(54) **HEXAGONAL BORON NITRIDE POWDER FOR COSMETIC, AND COSMETIC**

(30) Priority: 25.03.2022 JP 2022049890
(71) Applicant: JFE Mineral & Alloy Company, Ltd., Tokyo 105-0014 (JP)
(72) Inventor: NAKAGAWA Yumi, Tokyo 105-0014 (JP); NABESHIMA Seiji, Tokyo 105-0014 (JP); KATAOKA Mizuki, Tokyo 105-0014 (JP)
(74) Representative: Hoffmann Eitle
(86) International application number: PCT/JP2023/011317
(87) International publication number: WO 2023/182380

(57) **Abstract**

A hexagonal boron nitride powder for cosmetic use that provides low hiding power, excellent smoothness, and excellent conformability to the skin is provided, and a cosmetic in which the hexagonal boron nitride powder is used is provided.

A hexagonal boron nitride powder for cosmetic use includes primary particles having a scalelike shape and includes secondary particles formed of a stack of other primary particles having a scalelike shape. In the hexagonal boron nitride powder, an average particle size is 4 to 15 um, an aspect ratio is 30 or less, an average thickness of the primary particles and the other primary particles is 0.3 to 0.7 um, and a number of the secondary particles is 40% or less of a total number of the primary particles and the secondary particles. An oxygen concentration may be 0.5 mass% or less. Furthermore, a cosmetic includes the hexagonal boron nitride powder.

## Description

### Technical Field

The present invention relates to a hexagonal boron nitride powder for cosmetic use and to a cosmetic in which the hexagonal boron nitride powder is used.

### Background Art

Hexagonal boron nitride (hereinafter also referred to simply as "boron nitride" or abbreviated as "BN") is widely used as a pigment for cosmetics (cosmetic products).

For example, Patent Literature 1 discloses a hexagonal boron nitride powder in which side surfaces of the powder, which has a flat shape, are facet surfaces formed of six crystal faces corresponding to the (100) plane and six crystal faces corresponding to the (110) plane, as described in a hexagonal representation.

Furthermore, Patent Literature 2 discloses a boron nitride powder for cosmetic use that is formed of flattened-shape aggregates that are stacks of primary particles, the primary particles having a flattened shape with an average long diameter of 2 to 20 um and a thickness of 0.05 to 0.5 um. In the boron nitride powder, a specific surface area is 1 to 10 m²/g, a content of aggregates that pass through a 45-um mesh sieve is 50 mass% or greater, and an amount of soluble boron is 100 ppm or less.

Furthermore, Patent Literature 3 discloses a boron nitride powder for cosmetic use that is formed of aggregates of primary particles having a scalelike shape with an average long diameter of 4 to 15 um, a thickness of 0.2 to 0.7 um, and an aspect ratio of greater than 20. In the boron nitride powder, a specific surface area is 2 to 8 m²/g, and an amount of soluble boron is 100 ppm or less.

### Citation List

### Patent Literature

PTL 1: Japanese Unexamined Patent Application Publication No. 5-186205
PTL 2: Japanese Unexamined Patent Application Publication No. 2012-176910
PTL 3: Japanese Unexamined Patent Application Publication No. 2015-140337

### Summary of Invention

### Technical Problem

Regarding modern cosmetics, there is a need for them to have an ability to provide a natural-looking skin appearance rather than coverage, and, therefore, low hiding power is preferred. Furthermore, there is a need for an ability to give a feel of spreading lightly when they are applied to the skin, and, therefore, high smoothness is preferred. Furthermore, there is a need for cosmetics that provide proper wear comfort, that is, a good fit to the skin, without providing a thickly made-up appearance, which may result if they have excessively strong adhesion; in other words, proper conformability to the skin are preferred.

Unfortunately, the hexagonal boron nitride powders disclosed in Patent Literature 1 to 3, listed above, cannot be considered to satisfy all of the properties, namely, low hiding power, smoothness, and conformability to the skin.

The present invention has been made in view of the problem, and objects of the present invention are to provide a hexagonal boron nitride powder for cosmetic use that provides low hiding power, excellent smoothness, and excellent conformability to the skin, in instances in which the powder is used in a cosmetic, and to provide a cosmetic in which the hexagonal boron nitride powder is used.

As used herein, the expression "low hiding power" refers to a hiding power of 16 or less, and the hiding power is a value determined in the following manner. A hexagonal boron nitride powder is applied to an area of 2 cm × 2 cm of a black artificial leather with a nylon bristle flat brush (bristle length: 15 mm, ferrule width: 1.1 cm), and a whiteness is measured. The measurement and the removal of the surface deposit with a brush are repeated, and a point at which a change in the value of the whiteness becomes zero is designated as an endpoint. The value obtained by subtracting the whiteness of the artificial leather (18.6) from the whiteness at the endpoint is designated as the hiding power.

Furthermore, the expression "excellent smoothness" refers to a coefficient of dynamic friction of 0.31 or less, as determined in the following manner, by using a Trilab Handy Tribo-master TL201Ts (manufactured by Trinity-Lab. Inc.). 0.05 g of a hexagonal boron nitride powder is placed on an artificial leather, reciprocation is performed 20 times over a reciprocating distance of 50 mm at a load of 30 g and a moving speed of 10 mm/s, and the coefficient of dynamic friction on the 20th reciprocating motion is measured.

Furthermore, the expression "excellent conformability to the skin" refers to a skin conformability index of 0.04 to 0.08, as determined in the following manner, by using a Trilab Handy Tribo-master TL201Ts (manufactured by Trinity-Lab. Inc.). 0.05 g of a hexagonal boron nitride powder is placed on an artificial leather, reciprocation is performed twice over a reciprocating distance of 50 mm at a load of 30 g and a moving speed of 10 mm/s, the coefficient of dynamic friction is measured, and the difference between the coefficients of dynamic friction on the first and second runs is determined as the skin conformability index. Solution to Problem

The present inventors diligently conducted studies regarding the above-described problem and, consequently, discovered that when a ratio of the number of secondary particles (agglomerates) is not greater than a predetermined ratio, a condition with low hiding power, excellent smoothness, and excellent conformability to the skin can be achieved; the secondary particles are particles formed of primary particles (crystals and substrate particles) of the hexagonal boron nitride powder stacked on top of each other.

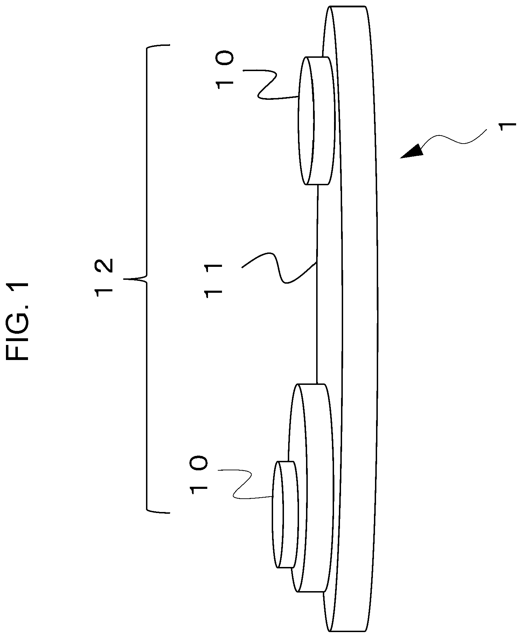

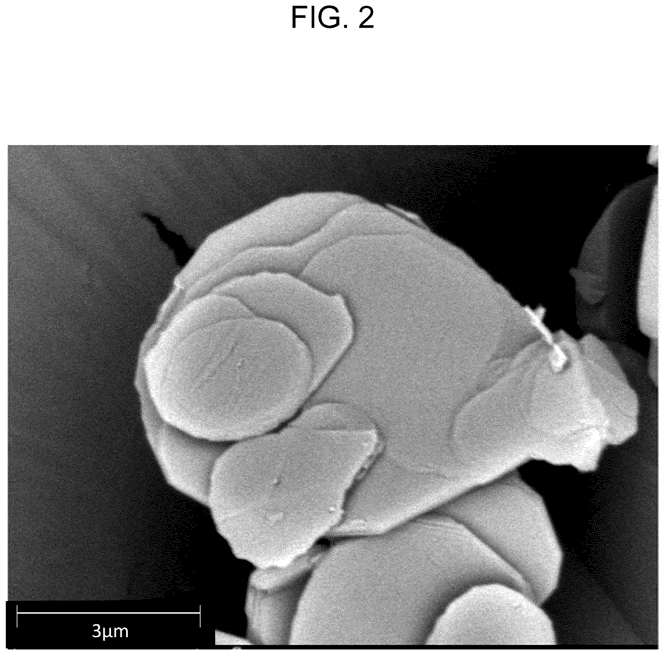

Fig. 1 is a schematic diagram illustrating primary particles 10 and 11 and a secondary particle 12 of a hexagonal boron nitride powder 1. Fig. 2 is an electron micrograph of the hexagonal boron nitride powder; the electron micrograph is used to describe the primary particle and the secondary particle. As illustrated in Fig. 1, one or more small particles 10 are stacked on a surface of a flat-shaped primary particle (large particle) 11, and thus, the secondary particle 12, which is a stepped stack, is formed. An example of the secondary particle that is a stepped stack is shown in the electron micrograph of Fig. 2. The present inventors discovered that it is possible to achieve a ratio of the secondary particles 12 that is not greater than a predetermined ratio by controlling the conditions for heat-treating crude BN from which the hexagonal boron nitride powder 1 is prepared.

As referred to in the present invention, the "secondary particle" is a particle formed of a primary particle (large particle) and a primary particle (small particle) stacked thereon. The primary particle (large particle) has a particle size of 3 to 20 um. The primary particle (small particle) has a particle size of 10 to 80% of the particle size of the primary particle (large particle).

If a primary particle (small particle) has a particle size greater than 80% of the particle size of the primary particle (large particle), the primary particle (small particle) can be regarded as the same as the primary particle (large particle) and is, therefore, not a primary particle (small particle).

For example, when a large particle has a particle size of 3 um, the particle size of a small particle is 0.3 to 2.4 um, and when a large particle has a particle size of 20 um, the particle size of a small particle is 2 to 16 um.

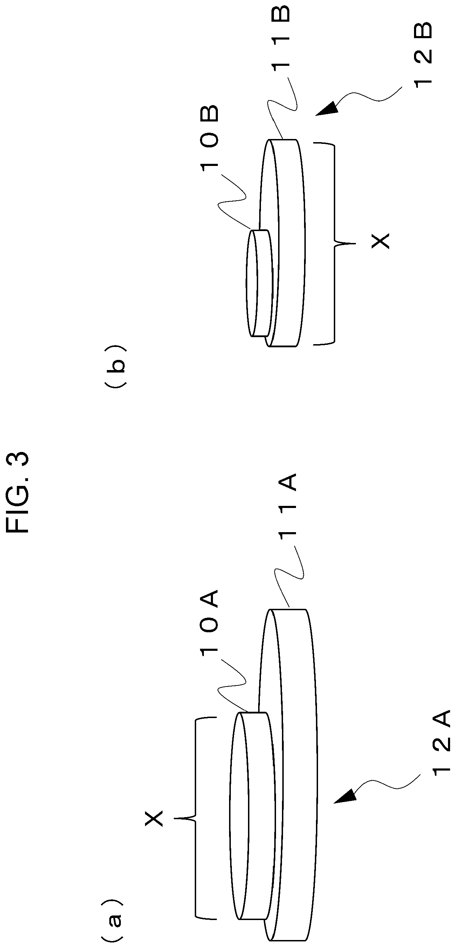

Fig. 3 is a schematic diagram illustrating primary particles (large particles) and primary particles (small particles).

Fig. 3(a) illustrates an example of a secondary particle 12A, which has a relatively large particle size, and Fig. 3(b) illustrates an example of a secondary particle 12B, which has a relatively small particle size.

The secondary particle 12A is formed of a primary particle (large particle) 11A and a primary particle (small particle) 10A stacked thereon. The secondary particle 12B is formed of a primary particle (large particle) 11B and a primary particle (small particle) 10B stacked thereon. In this instance, if the condition that the particle size of the primary particle (small particle) is 10 to 80% of the particle size of the primary particle (large particle) is satisfied as described above, the range of the particle size of the primary particle (small particle) is not limited, and the range of the particle size of the primary particle (large particle) is also not limited. That is, for example, the primary particle (small particle) 10A and the primary particle (large particle) 11B may be particles having the same or substantially the same size, for instance, having a particle size of X; accordingly, in the secondary particle 12A, which has a relatively large particle size, the primary particle 10A can be a small particle, and, in the secondary particle 12B, which has a relatively small particle size, the primary particle 11B can be a large particle.

A secondary particle is a particle formed of a primary particle (large particle) and one or more primary particles (small particles) stacked thereon, as described above, and, accordingly, a particle formed of one primary particle (large particle) and multiple primary particles (small particles) stacked thereon is regarded as one secondary particle.

Neither a particle formed of a primary particle (large particle) and another primary particle (large particle) stacked thereon nor a particle formed of a primary particle (small particle) and another primary particle (small particle) stacked thereon is regarded as a secondary particle. A stack formed of a primary particle (large particle) and another primary particle (large particle) is regarded as two primary particles, and a stack formed of a primary particle (small particle) and another primary particle (small particle) is also regarded as two primary particles.

The size of the primary particles is determined by performing observation with an SEM (magnification: 4000×). For the secondary particles, the preparation is performed as follows. The hexagonal boron nitride powder is applied with a brush, and the flat surface of the hexagonal boron nitride powder is brought into close contact with an artificial leather. Then, air is blown through an air dryer to remove extra powder to create a state in which powder particles do not overlap one another. Thereafter, the number of particles whose state of stacking can be observed and which can be clearly determined to be in the form of a stack is measured.

The present invention was made based on the above-described discoveries and are as follows.
[1] A hexagonal boron nitride powder for cosmetic use including:
   primary particles having a scalelike shape; and
   secondary particles formed of a stack of other primary particles having a scalelike shape, wherein
   an average particle size is 4 to 15 um,
   an aspect ratio is 30 or less,
   an average thickness of the primary particles and the other primary particles is 0.3 to 0.7 um, and
   a number of the secondary particles is 40% or less of a total number of the primary particles and the secondary particles.
[2] The hexagonal boron nitride powder for cosmetic use according to [1], wherein an oxygen concentration is 1.0 mass% or less.
[3] A cosmetic including the hexagonal boron nitride powder according to [1] or [2].

### Advantageous Effects of Invention

In accordance with the present invention, a hexagonal boron nitride powder for cosmetic use that provides low hiding power, excellent smoothness, and excellent conformability to the skin is provided, and a cosmetic in which the hexagonal boron nitride powder is used is provided.

### Brief Description of Drawings

[Fig. 1] Fig. 1 is a schematic diagram illustrating primary particles and a secondary particle of a hexagonal boron nitride powder.
[Fig. 2] Fig. 2 is an electron micrograph of the hexagonal boron nitride powder; the electron micrograph is used to describe primary particles and secondary particles.
[Fig. 3] Fig. 3 is a schematic diagram illustrating primary particles (large particles) and primary particles (small particles).

### Description of Embodiments

Now, a hexagonal boron nitride powder of the present invention, a method for producing the same, and a cosmetic in which the hexagonal boron nitride powder is used will be described in order.

### <Hexagonal Boron Nitride Powder>

A hexagonal boron nitride powder of the present invention is a hexagonal boron nitride powder for cosmetic use. The hexagonal boron nitride powder includes primary particles having a scalelike shape and includes secondary particles. In the hexagonal boron nitride powder, an average particle size is 4 to 15 um, an aspect ratio is 30 or less, an average thickness of the primary particles is 0.3 to 0.7 um, and the number of the secondary particles is 40% or less of the total number of the primary particles and the secondary particles.

### (Average Particle Size: 4 to 15 um)

If the average particle size of the hexagonal boron nitride powder is less than 4 um, a white color is exhibited, that is, transparency is reduced, in instances in which the powder is used in a cosmetic. On the other hand, if the average particle size of the powder is greater than 15 um, the skin may not feel smooth. Accordingly, the average particle size of the hexagonal boron nitride powder is specified to be 4 to 15 um. Preferably, the average particle size is greater than or equal to 5 um. Furthermore, preferably, the average particle size is less than or equal to 12 um. As referred to in the present invention, the average particle size of the hexagonal boron nitride powder is an average particle size of the powder in which the secondary particles and the primary particles are mixed with each other. The average particle size can be measured by a method described later in the Examples section.

### (Aspect Ratio: 30 or less)

If the aspect ratio is less than 7, an excessively high hiding power may be exhibited in instances in which the powder is used in a cosmetic. Accordingly, the aspect ratio is preferably greater than or equal to 7. On the other hand, if the aspect ratio is greater than 30, the skin may not feel smooth. Accordingly, the aspect ratio is specified to be less than or equal to 30. Preferably, the aspect ratio is greater than or equal to 7. More preferably, the aspect ratio is greater than or equal to 10. Even more preferably, the aspect ratio is greater than or equal to 14. Furthermore, preferably, the aspect ratio is less than or equal to 20. The aspect ratio is a value (average particle size/average thickness) obtained by dividing the average particle size (um), described above, of the powder by the average thickness (um), described below, of the primary particles. The aspect ratio can be measured by a method described later in the Examples section.

### (Average Thickness of Primary Particles: 0.3 to 0.7 um)

Since the average particle size of the powder is to be 4 to 15 um as described above, a hexagonal boron nitride powder having an average thickness of primary particles of less than 0.3 um is difficult to produce. On the other hand, if the average thickness of the primary particles is greater than 0.7 um, the skin may not feel smooth in instances in which the powder is used in a cosmetic. Accordingly, the average thickness of the primary particles is specified to be 0.3 to 0.7 um. The average thickness is preferably 0.3 to 0.6 um and more preferably 0.3 to 0.5 um. The average thickness of the primary particles can be measured by a method described later in the Examples section.

### (Ratio of Secondary Particles: 40% or less)

As referred to in the present invention, the secondary particle is a particle formed of a primary particle (large particle) having a particle size of 3 to 20 um and a primary particle (small particle) stacked thereon, and the particle size of the primary particle (small particle) is 10 to 80% of the particle size of the primary particle (large particle), as described above.

In the present invention, a ratio of the number of the secondary particles to the total number of the primary particles and the secondary particles (hereinafter also referred to simply as a "ratio of the secondary particles") is to be less than or equal to 40%. If the ratio of the secondary particles is greater than 40%, low hiding power, excellent smoothness, and excellent conformability to the skin cannot be exhibited in instances in which the powder is used in a cosmetic. Accordingly, in the present invention, the ratio of the secondary particles is specified to be less than or equal to 40%. It is preferable that the ratio of the secondary particles be as low as possible. The ratio is preferably less than or equal to 30% and more preferably less than or equal to 20%. The ratio of the secondary particles may be 0%, greater than or equal to 3%, or greater than or equal to 5%.

The ratio of the secondary particles can be measured by a method described later in the Examples section.

### (Oxygen Concentration: 1.0 mass% or less)

In the present invention, an oxygen concentration is not particularly limited and preferably less than or equal to 1.0 mass%. When the oxygen concentration is less than or equal to 1.0 mass%, adherence of the particles to each other can be inhibited to a greater degree, and, consequently, the particles can be deposited on the skin surface in a uniformly oriented manner. The oxygen concentration is more preferably less than or equal to 0.8 mass%, even more preferably less than or equal to 0.6 mass%, and still more preferably less than or equal to 0.5 mass%. The oxygen concentration is more preferably less than or equal to 0.4 mass% and even more preferably less than or equal to 0.3 mass%. The oxygen concentration may be 0 mass% or may be greater than or equal to 0.1 mass%.

### <Method for Producing Hexagonal Boron Nitride Powder>

Now, regarding a method for producing the hexagonal boron nitride powder of the present invention, preferred conditions will be described.

The method for producing the hexagonal boron nitride powder of the present invention is a method for producing the hexagonal boron nitride powder described above and includes a primary heating step and a secondary heating step, which is performed after the primary heating step. In the primary heating step, a crude boron nitride powder is heated to a first holding temperature of 1500 to 1900°C and then held at the first holding temperature for 1 to 5 hours, under an inert gas atmosphere. In the secondary heating step, the crude boron nitride powder is heated from the first holding temperature to a second holding temperature of 1950 to 2300°C and then held at the second holding temperature for 3 to 20 hours, under an inert gas atmosphere.

Note that the temperatures mentioned in the description below and the temperatures just mentioned are furnace temperatures.

First, a crude material, which is a crude boron nitride powder including a high-purity turbostratic boron nitride powder, is prepared.

The boron nitride powder can be prepared by uniformly mixing urea and/or a compound thereof (e.g., dicyandiamide or melamine) with boric acid and/or a dehydrated product thereof and then heating the mixture from 500°C to 1200°C in an inert gas atmosphere.

Regarding the mixing mentioned above, it is necessary that a ratio of nitrogen (N)/boron (B), in terms of a molar ratio, be 1 to 5. If the N/B, in terms of a molar ratio, is less than 1, an amount of impurities increases, which leads to excessive particle growth of the primary particles. On the other hand, if the N/B is greater than 5, sufficient particle growth cannot be expected because of the excessive amount of N.

Accordingly, regarding the mixing mentioned above, the nitrogen (N)/boron (B) ratio, in terms of a molar ratio, is specified to be 1 to 5.

With the heat treatment described above, it is possible to obtain a turbostratic boron nitride powder containing appropriate oxygen and carbon.

The "appropriate amounts of oxygen and carbon" are amounts that satisfy the following ranges: oxygen (0) in an amount of 10 to 40 mass%, carbon (C) in an amount of 0.01 to 10 mass%, and an oxygen (0)/carbon (C) ratio, in terms of a molar ratio, of 2.0 or greater.

Furthermore, in the present invention, the term "turbostratic structure" means that BN has a structure that is not fully crystallized, as represented by a broad peak, not a sharp peak of a hexagonal system, when analyzed by X-ray diffraction.

### (Two-Step Heating)

Next, a two-step heating is performed. This is to obtain a hexagonal boron nitride powder of the present invention, which has the ratio of the secondary particles of 40% or less, from the crude boron nitride powder.

The present inventors focused on the following facts: a boric acid compound present in impurities that are present in an amount of approximately 40 mass% in the crude material has a boiling point of approximately 1880°C; the tBN (turbostratic boron nitride powder) has a sublimation temperature of 2500°C or greater; and the liquid phase of the hexagonal boron nitride experiences a structural change at approximately 1400°C.

Accordingly, the holding temperatures were controlled in a stepwise manner, and as a result, the formation of the secondary particles that were to be included in the hexagonal boron nitride powder was inhibited, for an unknown reason.

Based on this discovery, it was discovered that by performing, on the crude boron nitride powder, two-step heating in which a first holding treatment and a second holding treatment are performed in a sequence in heat treatments with specific conditions, it is possible to obtain a hexagonal boron nitride powder having a ratio of the secondary particles of 40% or less.

### Primary Heating Step: First Heat Treatment and First Holding Treatment

In the primary heating step, the crude boron nitride powder is heated to the first holding temperature of 1500 to 1900°C and then held at the first holding temperature for 1 to 5 hours, under an inert gas atmosphere.

Specifically, the heat treatment (first heat treatment) to be performed is, for example, one in which a crude boron nitride powder prepared as described above is heated to the intermediate holding temperature (first holding temperature) of 1500 to 1900°C from ambient temperature (20 to 25°C) at a heating rate of 2 to 10°C/min, in an inert gas atmosphere at ambient or increased pressure. If the holding temperature is excessively low, crystal growth is not promoted, which results in an increased ratio of the secondary particles.

On the other hand, if the first holding temperature is excessively high, the first holding temperature is not significantly different from the second holding temperature, and, therefore, an effect of the two-step heating is unlikely to be produced; consequently, the ratio of the secondary particles in the resulting hexagonal boron nitride powder is substantially the same as the ratio that would result if the first holding treatment is not performed.

Accordingly, the first holding temperature is specified to be 1500 to 1900°C. Preferably, the first holding temperature is 1700 to 1900°C.

Subsequently, the resultant is held at the first holding temperature for 1 to 5 hours (first holding treatment). In either case of an excessively short holding time or an excessively long holding time, the ratio of the secondary particles increases. Preferably, the holding time associated with the first holding temperature is 3 to 5 hours.

### Secondary Heating Step: Second Heat Treatment and Second Holding Treatment

In the secondary heating step, which follows the primary heating step, the crude boron nitride powder is heated to the second holding temperature of 1950 to 2300°C from the first holding temperature and then held at the second holding temperature for 3 to 20 hours, under an inert gas atmosphere.

Specifically, the heat treatment (second heat treatment) to be performed is, for example, one in which, after the first holding (intermediate holding), the crude boron nitride powder is heated to the second holding temperature of 1950 to 2300°C at a heating rate of 1 to 5°C/min, in an inert gas atmosphere at ambient or increased pressure. Subsequently, the resultant is held at the second holding temperature for 3 to 20 hours (second holding treatment) and then cooled to room temperature (15 to 25°C). Preferably, the holding time associated with the second holding temperature is 6 hours or more. Furthermore, preferably, the holding time associated with the second holding temperature is 15 hours or less.

Note that in the secondary heating step, the heating (temperature increase) to the second holding temperature is started continuously after the primary heating step, without cooling the resultant to room temperature (15 to 25°C). Preferably, the heating to the second holding temperature is started immediately after the completion of the primary heating step.

Thereafter, the resulting boron nitride powder may be subjected to grinding and classification.

Thus, the hexagonal boron nitride powder for cosmetic use of the present invention can be prepared.

### <Cosmetic in Which Hexagonal Boron Nitride Powder is Used>

The hexagonal boron nitride powder of the present invention can be used as a pigment for cosmetic use in a variety of applications, such as those described below.

Specifically, the hexagonal boron nitride powder is suitable for use, for example, in make-up preparations, such as foundations, face powders, make-up bases, face colors, and blushers, and skin care preparations, such as sunscreen agents, creams, milky lotions, and toners.

Fundamental components of these cosmetics are not particularly limited and may be those known in the related art. The hexagonal boron nitride powder of the present invention can be used in place of a boron nitride powder or an inorganic powder in the components of the related art (examples of the inorganic powder include those of silicic anhydride, aluminum oxide, titanium oxide, zinc oxide, zirconium oxide, mica, or talc).

### EXAMPLES

Examples of the present invention will be described below.

### <Example 1>

Melamine and boric acid were mixed together in a mass ratio of 1:1, and the mixture was heated to 700°C in a N₂ atmosphere at 1 atm and held at 700°C for 10 hours. The resulting crude boron nitride (crude BN) was ground, and the resultant was analyzed by laser light diffraction (dry, 3.0 bar) and confirmed to have an average particle size of 4 um.

Next, 5 kg of the crude boron nitride (crude BN) was placed into a graphite crucible with a lid and then heated to 1880°C at a heating rate of 4°C/min (first heat treatment). Subsequently, the resultant was held at 1880°C for 3 hours (first holding treatment). Subsequently, the resultant was heated to 2000°C at a heating rate of 4°C/min (second heat treatment) and then held at 2000°C for 10 hours in a N₂ atmosphere. Subsequently, the resultant was cooled to room temperature (20°C), also in a N₂ atmosphere. Thus, a hexagonal boron nitride powder (BN powder) was prepared. After the cooling, the hexagonal boron nitride powder was subjected to grinding and classification. The resulting product was identified with an X-ray diffractometer and was confirmed to be a highly crystallized boron nitride powder.

### <Example 2>

A hexagonal boron nitride powder was prepared under the same conditions as in Example 1, except that the holding time for the first holding treatment was 5 hours instead of 3 hours, as compared to Example 1.

### <Example 3>

A hexagonal boron nitride powder was prepared under the same conditions as in Example 1, except that the heating temperature for the first heat treatment (first holding temperature) was 1860°C instead of 1880°C, as compared to Example 1.

### <Example 4>

A hexagonal boron nitride powder was prepared under the same conditions as in Example 2, except that the heating temperature for the first heat treatment (first holding temperature) was 1860°C instead of 1880°C, as compared to Example 2.

### <Example 5>

A hexagonal boron nitride powder was prepared under the same conditions as in Example 2, except that the holding time for the second holding treatment was 8 hours instead of 10 hours, as compared to Example 2.

### <Example 6>

A hexagonal boron nitride powder was prepared under the same conditions as in Example 2, except that the heating temperature for the second heat treatment (second holding temperature) was 1970°C instead of 2000°C and that the holding time for the second holding treatment was 6 hours instead of 10 hours, as compared to Example 2.

### <Example 7>

A hexagonal boron nitride powder was prepared under the same conditions as in Example 2, except that the heating temperature for the second heat treatment (second holding temperature) was 1950°C instead of 2000°C and that the holding time for the second holding treatment was 4 hours instead of 10 hours, as compared to Example 2.

### <Example 8>

A hexagonal boron nitride powder was prepared under the same conditions as in Example 2, except that the heating temperature for the second heat treatment (second holding temperature) was 1950°C instead of 2000°C and that the holding time for the second holding treatment was 3 hours instead of 10 hours, as compared to Example 2.

### <Example 9>

A hexagonal boron nitride powder was prepared under the same conditions as in Example 2, except that the heating temperature for the first heat treatment (first holding temperature) was 1600°C instead of 1880°C, that the heating temperature for the second heat treatment (second holding temperature) was 2050°C instead of 2000°C, and that the holding time for the second holding treatment was 5 hours instead of 10 hours, as compared to Example 2.

### <Comparative Example 1>

Melamine and boric acid were mixed together in a mass ratio of 1:1, and the mixture was heated to 700°C in a N₂ atmosphere at 1 atm and held at 700°C for 10 hours. The resulting crude boron nitride (crude BN) was ground, and the resultant was confirmed, in a manner similar to that of Example 1, to have an average particle size of 4 um.

Next, 5 kg of the crude boron nitride (crude BN) was placed into a graphite crucible with a lid and then heated to 1880°C at a heating rate of 4°C/min in a N₂ atmosphere. Subsequently, the resultant was held at 1880°C for 3 hours in a N₂ atmosphere and subsequently cooled to room temperature in a N₂ atmosphere over a period of 5 hours or more.

Thereafter, the resultant was subjected to grinding, classification, and washing and subsequently held at 2000°C for 10 hours, also in a N₂ atmosphere. Thereafter, the resultant was cooled to room temperature. Subsequently, the resulting boron nitride powder was subjected to grinding and classification to give a hexagonal boron nitride powder.

### <Comparative Example 2>

Melamine and boric acid were mixed together in a mass ratio of 1:1, and the mixture was heated to 700°C in a N₂ atmosphere at 1 atm and held at 700°C for 10 hours. The resulting crude boron nitride (crude BN) was ground, and the resultant was confirmed, in a manner similar to that of Example 1, to have an average particle size of 4 um.

Next, 5 kg of the crude boron nitride (crude BN) was placed into a graphite crucible with a lid and then heated to 2000°C at a heating rate of 4°C/min in a N₂ atmosphere. Subsequently, the resultant was held at 2000°C for 10 hours in a N₂ atmosphere and subsequently cooled to room temperature, also in a N₂ atmosphere. The resulting boron nitride powder was subjected to grinding, classification, and washing to remove impurities, thereby increasing purity to give a hexagonal boron nitride powder.

### <Comparative Example 3>

A hexagonal boron nitride powder was prepared under the same conditions as in Comparative Example 2, except that the heating temperature for the heat treatment of the crude boron nitride (crude BN) was 1750°C instead of 2000°C and that the holding time for the holding treatment was 5 hours instead of 10 hours, as compared to Comparative Example 2.

### <Comparative Example 4>

A hexagonal boron nitride powder was prepared under the same conditions as in Comparative Example 2, except that the heating temperature for the heat treatment of the crude boron nitride (crude BN) was 2040°C instead of 2000°C and that the holding time for the holding treatment was 9 hours instead of 10 hours, as compared to Comparative Example 2.

### <Comparative Example 5>

Melamine and boric acid were mixed together in a mass ratio of 1:1, and the mixture was heated to 700°C in a N₂ atmosphere at 1 atm and held at 700°C for 10 hours. The resulting crude boron nitride (crude BN) was ground, and the resultant was analyzed by laser light diffraction (dry, 3.0 bar) and confirmed to have an average particle size of 4 um.

Next, 5 kg of the crude boron nitride (crude BN) was placed into a graphite crucible with a lid and then heated to 1450°C at a heating rate of 4°C/min (first heat treatment). Subsequently, the resultant was held at 1450°C for 10 hours (first holding treatment). Subsequently, the resultant was heated to 2050°C at a heating rate of 4°C/min (second heat treatment) and then held at 2000°C for 2 hours in a N₂ atmosphere. Subsequently, the resultant was cooled to room temperature (20°C), also in a N₂ atmosphere. The resulting boron nitride powder was subjected to grinding, classification, and washing to remove impurities, thereby increasing purity to give a hexagonal boron nitride powder.

### <Comparative Example 6>

A hexagonal boron nitride powder was prepared under the same conditions as in Comparative Example 2, except that the heating temperature for the heat treatment of the crude boron nitride (crude BN) was 2100°C instead of 2000°C and that the holding time for the holding treatment was 9 hours instead of 10 hours, as compared to Comparative Example 2.

Table 1 shows the measurement results of the average thickness of the primary particles, the average particle size, the oxygen concentration, the aspect ratio, and the ratio of the secondary particles, of the hexagonal boron nitride powders prepared under the respective production conditions.

The average thickness, the average particle size, the oxygen concentration, the aspect ratio, and the ratio of the secondary particles were measured in the following manners.

### (Average Thickness of Primary Particles)

The prepared hexagonal boron nitride powder (BN powder) was subjected to secondary electron observation, which was performed with an SEM (magnification: 20000×, acceleration voltage: 10 kV). Of the primary particles having a scalelike shape, those having a flat portion that forms an angle of 75 to 90 degrees with the observation surface were selected for the measurement of the thickness. The thickness of randomly selected 30 particles was measured, and an average of the thicknesses (sum of the thicknesses of the 30 particles/30) was designated as the average thickness.

Regarding the primary particles, both the primary particles (large particles) and the primary particles (small particles) were the targets of the measurement of the average thickness.

Furthermore, even in instances where primary particles formed a stack, each of the primary particles was measured to determine the average thickness.

### (Average Particle Size)

The hexagonal boron nitride powder was added to a surfactant (nonionic surfactant), which had been diluted to 0.25 mass% with water, and dispersed in an ultrasonic cleaning machine. Subsequently, a light scattering pattern obtained by projecting a laser beam onto the BN sample was analyzed, and the particle size distribution was measured (with a particle size distribution analyzer (Mastersizer 3000, manufactured by Spectris Co., Ltd.) in a dry mode) to determine the average particle size.

### (Aspect Ratio)

The average particle size (um), which was determined by the above-described measurement method, was divided by the average thickness (um), which was in accordance with the above-described measurement method, and the resulting value was designated as the aspect ratio.

### (Oxygen Concentration)

10 mg of the sample was placed in an oxygen-nitrogen analyzer, and a measurement was performed with an inert gas-impulse heat melting method and a non-dispersive infrared absorption method.

### (Ratio of Secondary Particles)

The hexagonal boron nitride powder was applied to an artificial leather (Supplale (registered trademark)) with a brush, and the flat surface of the hexagonal boron nitride powder was brought into close contact with the artificial leather. Then, air was blown through an air dryer to remove extra powder to create a state in which powder particles did not overlap one another. A surface of the particles of the powder was observed with an SEM (magnification: 4000×, acceleration voltage: 10 kV, secondary electron), and a total of 100 particles, which were randomly selected and included primary particles and secondary particles, was counted to calculate the ratio of the secondary particles.

### [Evaluation]

The hexagonal boron nitride powders prepared under the respective production conditions were evaluated as follows.

### <Hiding Power>

The hexagonal boron nitride powder was applied to an area of 2 cm × 2 cm of an artificial leather (Supplale (registered trademark)) (black), with a nylon bristle flat brush (bristle length: 15mm, ferrule width: 1.1 cm), and the whiteness was measured with a Konica Minolta CR-14 colorimeter on the Hunter scale. A cycle of "the measurement, the removal of the surface deposit with a brush, and the measurement" was repeated, and a point at which a change in the value of the whiteness became zero was designated as an endpoint. The value obtained by subtracting the whiteness of the artificial leather (18.6) from the whiteness at the endpoint was designated as the hiding power.

In instances where the value was 16 or less, it was determined that the hiding power was sufficiently low, and, therefore, a rating of "Pass" was given.

### <Coefficient of Dynamic Friction>

0.05 g of the hexagonal boron nitride powder was placed on an artificial leather, and the coefficient of dynamic friction was measured with a Trilab Handy Tribo-master TL201Ts (manufactured by Trinity-Lab. Inc.). Specifically, reciprocation was performed 20 times over a reciprocating distance of 50 mm (25 mm for each of advance and return movements) at a load of 30 g and a moving speed of 10 mm/s. A fingerprint tactile contactor and an artificial leather (Supplale (registered trademark)) as a substrate were used. The measured value on the 20th reciprocating motion was designated as the coefficient of dynamic friction of the evaluation target. In instances where the coefficient of dynamic friction was 0.31 or less, it was determined that the smoothness was excellent, and, therefore, a rating of "Pass" was given.

### <Conformability to The Skin>

0.05 g of the hexagonal boron nitride powder was placed on an artificial leather, and the coefficient of dynamic friction was measured with a Trilab Handy Tribo-master TL201Ts (manufactured by Trinity-Lab. Inc.). Specifically, reciprocation was performed twice over a reciprocating distance of 50 mm at a load of 30 g and a moving speed of 10 mm/s. A fingerprint tactile contactor and an artificial leather (Supplale (registered trademark)) as a substrate were used. The difference between the coefficients of dynamic friction on the first and second runs was designated as the skin conformability index.

In instances where the skin conformability index was greater than 0.08, it was determined that an excessively strong adhesion would lead to a thickly made-up appearance. In instances where the skin conformability index was less than 0.04, it was determined that a good fit to the skin was not achieved. In instances where the skin conformability index was 0.04 to 0.08, it was determined that excellent conformability to the skin were achieved, and, therefore, a rating of "Pass" was given.

### <Feel of Skin>

The hexagonal boron nitride powder was applied to the skin of humans, and the feel of the skin was evaluated by six evaluators according to the following criteria.

### Evaluation Criteria

A: 5 or more and 6 or fewer of the evaluators answered that the skin felt smooth.
B: 3 or more and 4 or fewer of the evaluators answered that the skin felt smooth.
C: 2 or fewer of the evaluators answered that the skin felt smooth.

**[Table 1]**

| No. | Production Conditions | | | | | | | Average Particle Size (µm) | Average Thickness of Primary Particles (µm) | Oxygen Concentration (mass%) | Aspect Ratio | Ratio of Secondary Particles (%) | Properties | | | |
|---|---|---|---|---|---|---|---|---|---|---|---|---|---|---|---|---|
| | Primary Heating Step | | | Cooling to Room Temperature | Secondary Heating Step | | | | | | | | Coefficient of Dynamic Friction | Hiding Power | Skin Conformability Index | Feel of Skin |
| | Heating Temperature (First Holding Temperature) (°C) | Holding Time (hr) | Atmosphere | Yes/No | Heating Temperature (Second Holding Temperature) (°C) | Holding Time (hr) | Atmosphere | | | | | | | | | |
| Example 1 | 1880 | 3 | N₂ | No | 2000 | 10 | N₂ | 6.1 | 0.41 | 0.2 | 15 | 32 | 0.31 | 15.5 | 0.057 | A |
| Example 2 | 1880 | 5 | N₂ | No | 2000 | 10 | N₂ | 5.6 | 0.35 | 0.2 | 16 | 20 | 0.29 | 14.8 | 0.047 | A |
| Example 3 | 1860 | 3 | N₂ | No | 2000 | 10 | N₂ | 5.8 | 0.39 | 0.2 | 15 | 25 | 0.29 | 15.0 | 0.049 | A |
| Example 4 | 1860 | 5 | N₂ | No | 2000 | 10 | N₂ | 6.2 | 0.42 | 0.2 | 15 | 18 | 0.27 | 14.5 | 0.045 | A |
| Example 5 | 1880 | 5 | N₂ | No | 2000 | 8 | N₂ | 5.5 | 0.34 | 0.4 | 16 | 25 | 0.30 | 15.1 | 0.052 | A |
| Example 6 | 1880 | 5 | N₂ | No | 1970 | 6 | N₂ | 5.5 | 0.32 | 0.6 | 17 | 29 | 0.30 | 15.2 | 0.059 | A |
| Example 7 | 1880 | 5 | N₂ | No | 1950 | 4 | N₂ | 5.4 | 0.32 | 0.8 | 17 | 34 | 0.31 | 15.6 | 0.060 | A |
| Example 8 | 1880 | 5 | N₂ | No | 1950 | 3 | N₂ | 5.2 | 0.31 | 1.2 | 17 | 40 | 0.31 | 15.8 | 0.078 | A |
| Example 9 | 1600 | 5 | N₂ | No | 2050 | 5 | N₂ | 5.0 | 0.68 | 0.5 | 7 | 39 | 0.30 | 15.9 | 0.076 | B |
| Comparative Example 1 | 1880 | 3 | N₂ | Yes | 2000 | 10 | N₂ | 6.3 | 0.39 | 0.2 | 16 | 89 | 0.39 | 19.3 | 0.030 | A |
| Comparative Example 2 | - | - | - | No | 2000 | 10 | N₂ | 10.0 | 0.56 | 0.2 | 18 | 61 | 0.33 | 17.9 | 0.147 | B |
| Comparative Example 3 | - | - | - | No | 1750 | 5 | N₂ | 3.3 | 0.30 | 2.0 | 11 | 85 | 0.55 | 23.0 | 0.160 | A |
| Comparative Example 4 | - | - | - | No | 2040 | 9 | N₂ | 22.5 | 0.87 | 0.3 | 26 | 52 | 0.32 | 12.1 | 0.021 | C |
| Comparative Example 5 | 1450 | 10 | N₂ | No | 2050 | 2 | N₂ | 5.7 | 0.89 | 1.5 | 6 | 55 | 0.40 | 20.1 | 0.030 | C |
| Comparative Example 6 | - | - | - | No | 2100 | 9 | N₂ | 29.6 | 0.89 | 0.3 | 33 | 51 | 0.32 | 11.9 | 0.026 | C |

| | | | | | | | | | | | | | | | | |
|---|---|---|---|---|---|---|---|---|---|---|---|---|---|---|---|---|
| *The underline means falling outside the scope. *The symbol "-" regarding the primary heating step indicates that the primary heating was not performed. | | | | | | | | | | | | | | | | |

As shown in Table 1, the hexagonal boron nitride powders of Examples 1 to 9, in which the ratio of the secondary particles was 40% or less, had a coefficient of dynamic friction of 0.31 or less, which indicated that they had excellent smoothness. Furthermore, the hexagonal boron nitride powders of Examples 1 to 9 had a skin conformability index of 0.04 to 0.08, which indicated that they had excellent conformability to the skin. Furthermore, the hexagonal boron nitride powders of Examples 1 to 9 had a hiding power of 16 or less, which indicated that they had sufficiently low hiding power.

Thus, it was found that the hiding power, smoothness, and conformability to the skin were all excellent in Examples 1 to 9.

Furthermore, regarding the result of evaluation of the feel of the skin, while Comparative Examples 1 to 6 were rated as C, Examples 1 to 9, in which the average particle size was 4 to 15 um, and the average thickness of the primary particles was 0.3 to 0.7 um, were rated as A or B. In particular, in Examples 1 to 8, in which the average thickness of the primary particles was within a range of 0.3 to 0.5 um, the result of evaluation of the feel of the skin was A.

Furthermore, in Examples 1 to 8, in which the aspect ratio was 10 or greater, a hiding power of 15.8 or less was achieved, that is, a further reduced hiding power was achieved.

In overall view of low hiding power, excellent smoothness, and excellent conformability to the skin that are sought by the present invention, a particularly low hiding power and better smoothness and conformability to the skin were achieved in Examples 2 and 4, among Examples 1 to 9.

Specifically, in Examples 2 and 4, in which the ratio of the secondary particles was 20% or less, particularly high smoothnesses were achieved compared to other Examples, as indicated by the coefficients of dynamic friction of 0.29 and 0.27, particularly low hiding powers were achieved, as indicated by the hiding powers of 14.8 and 14.5, and particularly good conformability to the skin were achieved as indicated by the skin conformability indices of 0.047 and 0.045.

Furthermore, a comparison between Example 2 and Example 4 indicates that a lower hiding power, a better smoothness, and better conformability to the skin were achieved in Example 4, in which the ratio of the secondary particles was lower.

Furthermore, attention was focused on the oxygen concentration. Regarding Examples 2 and 5 to 8, which had the same conditions for production, except for the conditions of the secondary heating step, it was found that in Example 2, in which the oxygen concentration was 0.2 mass%, a higher smoothness was achieved as indicated by the lower coefficient of dynamic friction, and a particularly lower hiding power was achieved, than in Examples 5, 6, 7, and 8, in which the oxygen concentrations were 0.4 mass%, 0.6 mass%, 0.8 mass%, and 1.2 mass%, respectively. Furthermore, it was found that in Example 2, the conformability to the skin were better, as indicated by the skin conformability index of 0.047, than in Examples 5, 6, 7, and 8, in which the skin conformability indices were 0.052, 0.059, 0.060, and 0.078, respectively.

In contrast, in Comparative Example 1, the hexagonal boron nitride powder was prepared through a process including cooling to room temperature between steps corresponding to the primary heating step and the secondary heating step of the present invention; as a result, the ratio of the secondary particles was 89%, and thus, the desired hiding power, smoothness, and conformability to the skin could not be obtained.

Furthermore, in Comparative Example 2, the hexagonal boron nitride powder was prepared without performing treatments corresponding to the treatments of the primary heating step of the present invention; as a result, the ratio of the secondary particles was 61%, and thus, the desired hiding power, smoothness, and conformability to the skin could not be obtained.

Furthermore, in Comparative Example 3, the hexagonal boron nitride powder was prepared without performing treatments corresponding to the treatments of the primary heating step of the present invention and by using a temperature of 1750°C as a heating temperature corresponding to the heating temperature (holding temperature) of the secondary heating step of the present invention; as a result, the ratio of the secondary particles was 85%, the average particle size was 3.3 um, and thus, the desired hiding power, smoothness, and conformability to the skin could not be obtained.

Furthermore, in Comparative Example 4, the hexagonal boron nitride powder was prepared without performing treatments corresponding to the treatments of the primary heating step of the present invention; as a result, the ratio of the secondary particles was 52%, the average particle size was 22.5 um, the average thickness of the primary particles was 0.87 um, and thus, the desired smoothness and conformability to the skin could not be obtained.

Furthermore, in Comparative Example 5, the hexagonal boron nitride powder was prepared by using a temperature of 1450°C as a heating temperature corresponding to the heating temperature (first holding temperature) of the primary heating step of the present invention, using a time of 10 hours as a holding time corresponding to the holding time of the primary heating step, and using a time of 2 hours as a holding time corresponding to the holding time of the secondary heating step of the present invention; as a result, the ratio of the secondary particles was 55%, the average particle size was 2 um, the average thickness of the primary particles was 0.89 um, and thus, the desired hiding power, smoothness, and conformability to the skin could not be obtained.

Furthermore, in Comparative Example 6, the hexagonal boron nitride powder was prepared without performing treatments corresponding to the treatments of the primary heating step of the present invention; as a result, the ratio of the secondary particles was 51%, the average particle size was 29.6 um, the aspect ratio was 33, the average thickness of the primary particles was 0.89 um, and thus, the desired smoothness and conformability to the skin could not be obtained.

### Reference Signs List

- 1: hexagonal boron nitride powder
- 10: primary particle (small particle)
- 11: primary particle (large particle)
- 12: secondary particle

## Claims

1. A hexagonal boron nitride powder for cosmetic use comprising:
primary particles having a scalelike shape; and
secondary particles formed of a stack of other primary particles having a scalelike shape, wherein
an average particle size is 4 to 15 um,
an aspect ratio is 30 or less,
an average thickness of the primary particles and the other primary particles is 0.3 to 0.7 um, and
a number of the secondary particles is 40% or less of a total number of the primary particles and the secondary particles.

2. The hexagonal boron nitride powder for cosmetic use according to Claim 1, wherein an oxygen concentration is 1.0 mass% or less.

3. A cosmetic comprising the hexagonal boron nitride powder according to Claim 1 or 2.
